(19) Europäisches Patentamt
European Patent Office
Office européen des brevets

(11) EP 4 678 625 A1

(12) **EUROPEAN PATENT APPLICATION**
published in accordance with Art. 153(4) EPC

(43) Date of publication:
**14.01.2026 Bulletin 2026/03**

(21) Application number: **24770439.8**

(22) Date of filing: **20.02.2024**

(51) International Patent Classification (IPC):
***C07C 51/00*** *(2006.01)* ***B01F 23/43*** *(2022.01)*
***B01F 23/231*** *(2022.01)* ***B01F 27/90*** *(2022.01)*
***B01F 27/1122*** *(2022.01)* ***B01F 33/71*** *(2022.01)*
***B01F 35/221*** *(2022.01)* ***B01F 35/222*** *(2022.01)*
***B01J 31/24*** *(2006.01)* ***C07B 61/00*** *(2006.01)*
***C07C 53/06*** *(2006.01)*

(52) Cooperative Patent Classification (CPC):
**B01F 23/231; B01F 23/43; B01F 27/1122; B01F 27/90; B01F 33/71; B01F 35/221; B01F 35/222; B01J 31/24; C07B 61/00; C07C 51/00; C07C 53/06**

(86) International application number:
**PCT/JP2024/006111**

(87) International publication number:
**WO 2024/190326 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.03.2023 JP 2023037579**
**29.08.2023 JP 2023139078**

(71) Applicant: **Nitto Denko Corporation**
**Osaka 567-8680 (JP)**

(72) Inventors:
- **FUOKA, Daisuke**
  **Ibaraki-shi, Osaka 567-8680 (JP)**
- **MATSUDA, Hirokazu**
  **Ibaraki-shi, Osaka 567-8680 (JP)**

(74) Representative: **Hoffmann Eitle**
**Patent- und Rechtsanwälte PartmbB**
**Arabellastraße 30**
**81925 München (DE)**

(54) **METHOD FOR PRODUCING ORGANIC COMPOUND**

(57) A method for producing an organic compound according to the present invention is a method that includes reacting a starting compound using a catalyst in the presence of a gas phase and a liquid phase including an organic phase and an aqueous phase, thereby producing the organic compound. The method includes stirring the liquid phase under the stirring conditions where a stirring power per unit volume is 0.5 $kW/m^3$ or more and 3.5 $kW/m^3$ or less and a stirring blade tip speed is 1.10 m/s or more.

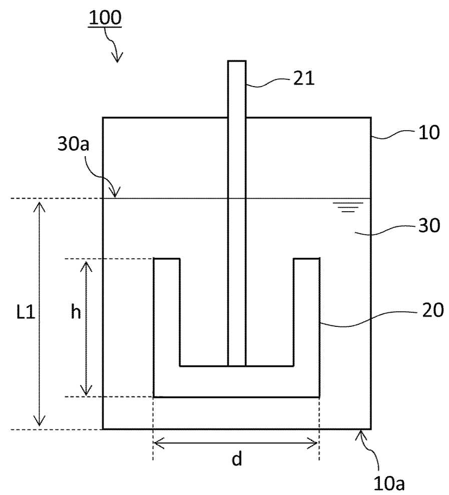


FIG.1

EP 4 678 625 A1

## Description

### TECHNICAL FIELD

The present invention relates to a method for producing an organic compound.

### BACKGROUND ART

Stirring conditions for efficient production of an organic compound have been studied. For example, Patent Literature 1 describes a method for producing aromatic cyanate esters in which a reaction is performed in a reaction vessel equipped with a stirrer under conditions where n $\times$ $d^{3/2}$ is 8 or more when the number of stirrings is n [rpm] and a stirring blade diameter is d [m].

### CITATION LIST

#### Patent Literature

Patent literature 1: JP 2000-128850A

### SUMMARY OF INVENTION

#### Technical Problem

The conventional stirring conditions may need some improvement. In particular, there is room for improvement in stirring conditions for a case of producing organic compounds in a reaction system that includes not only a liquid phase but also a gas phase.
Therefore, an object of the present invention is to provide a method for producing an organic compound, the method is suitable for improving the yield of the organic compound.

#### Solution to Problem

The present invention provides a method for producing an organic compound by reacting a starting compound using a catalyst in the presence of a gas phase and a liquid phase, the liquid phase including an organic phase and an aqueous phase,
the method including:
stirring the liquid phase under stirring conditions where a stirring power per unit volume is 0.5 kW/$m^3$ or more and 3.5 kW/$m^3$ or less and a stirring blade tip speed is 1.10 m/s or more.

#### Advantageous Effects of Invention

The present invention can provide a production method suitable for improving the yield of organic compounds.

### BRIEF DESCRIPTION OF DRAWINGS

FIG. 1 is a cross-sectional view schematically showing an example of a stirring device to be used in the method for producing an organic compound of the present embodiment.
FIG. 2 is a cross-sectional view of a stirring device, for explanation of an example of the method for producing an organic compound of the present embodiment.
FIG. 3 is a graph showing stirring conditions for Examples 1 to 12 and Comparative Examples 1 to 2.

### DESCRIPTION OF EMBODIMENTS

A method for producing an organic compound according to a first aspect of the present invention is a method for producing an organic compound by reacting a starting compound using a catalyst in the presence of a gas phase and a liquid phase, the liquid phase including an organic phase and an aqueous phase,
the method including:

stirring the liquid phase under stirring conditions where a stirring power per unit volume is 0.5 $kW/m^3$ or more and 3.5 $kW/m^3$ or less and a stirring blade tip speed is 1.10 m/s or more.

According to a second aspect of the present invention, for example, in the method for producing an organic compound according to the first aspect, the stirring blade tip speed is 1.8 m/s or more.

According to a third aspect of the present invention, for example, in the method for producing an organic compound according to the first or the second aspect, the stirring power per unit volume is 2.8 $kW/m^3$ or less.

According to a fourth aspect of the present invention, for example, in the method for producing an organic compound according to any one of the first to the third aspects, under the stirring conditions, the number of revolutions is 10 rpm or more and 1200 rpm or less.

According to a fifth aspect of the present invention, for example, in the method for producing an organic compound according to any one of the first to the fourth aspects, the liquid phase is 0.05 L or more and 7000 L or less.

According to a sixth aspect of the present invention, for example, in the method for producing an organic compound according to any one of the first to the fifth aspects, a proportion of the organic phase in the liquid phase is 5 vol% or more and 60 vol% or less.

According to a seventh aspect of the present invention, for example, in the method for producing an organic compound according to any one of the first to the sixth aspects, the liquid phase has a temperature of 40°C or higher and 100°C or lower in the reaction.

According to an eighth aspect of the present invention, for example, in the method for producing an organic compound according to any one of the first to the seventh aspects, the stirring is performed using a stirring blade, and in a static state, the stirring blade is in contact with an interface between the aqueous phase and the organic phase.

According to a ninth aspect of the present invention, for example, in the method for producing an organic compound according to any one of the first to the eighth aspects, the reaction is a reversible reaction.

According to a tenth aspect of the present invention, for example, in the method for producing an organic compound according to any one of the first to the ninth aspects, the organic phase includes the catalyst and the aqueous phase includes the starting compound.

According to an eleventh aspect of the present invention, for example, in the method for producing an organic compound according to any one of the first to the tenth aspects, the gas phase includes a gas capable of reacting with the starting compound.

According to a twelfth aspect of the present invention, for example, in the method for producing an organic compound according to the eleventh aspect, the gas includes hydrogen, and the reaction is a hydrogenation reaction of the starting compound.

According to a thirteenth aspect of the present invention, for example, in the method for producing an organic compound according to the twelfth aspect, the starting compound is at least one selected from the group consisting of carbon dioxide, a hydrogencarbonate and a carbonate, and a formate is synthesized from the starting compound by the reaction.

According to a fourteenth aspect of the present invention, for example, in the method for producing an organic compound according to any one of the first to the thirteenth aspects, the catalyst is a metal catalyst.

According to a fifteenth aspect of the present invention, for example, in the method for producing an organic compound according to the fourteenth aspect, the metal catalyst includes ruthenium.

According to a sixteenth aspect of the present invention, for example, in the method for producing an organic compound according to any one of the first to the fifteenth aspects, the organic phase includes toluene.

According to a seventeenth aspect of the present invention, for example, in the method for producing an organic compound according to any one of the first to the sixteenth aspects, the stirring power per unit volume is 0.5 $kW/m^3$ or more and 2.0 $kW/m^3$ or less.

According to an eighteenth aspect of the present invention, for example, in the method for producing an organic compound according to any one of the first to the seventeenth aspects, the stirring power per unit volume is 1.5 $kW/m^3$ or less.

The present invention will be described below in detail, though the following description is not intended to restrict the present invention to a specific embodiment.

(Method for producing organic compound)

The method for producing an organic compound of the present embodiment is a method for producing an organic compound by reacting a starting compound using a catalyst in the presence of a gas phase and also a liquid phase including an organic phase and an aqueous phase. The method for producing an organic compound of the present embodiment includes stirring the liquid phase under stirring conditions where stirring power per unit volume is 0.5 $kW/m^3$ or more and 3.5 $kW/m^3$ or less, and the stirring blade tip speed is 1.10 m/s or more. The aqueous phase is a phase including an aqueous solvent, the organic phase is a phase including an organic solvent, and the gas phase is a phase including a

gas. In the present description, an aqueous solvent and an organic solvent may be collectively referred to as a solvent. In the present description, the liquid phase including the organic phase and the aqueous phase may be referred to as a reaction liquid.

The stirring power per unit volume is a stirring power per volume of a liquid phase. The stirring blade tip speed is a value calculated by a mathematical formula: the number of revolutions n($s^{-1}$) $\times$ stirring blade diameter d(m) $\times$ $\pi$. The number of revolutions n is the number of revolutions of the stirring blade per second. The stirring blade diameter d is a diameter of a trajectory circle drawn by the blade tip.

In the production method of the present embodiment, the liquid phase is stirred to bring the gas phase into contact with the liquid phase such that the reaction of the starting compound proceeds. For example, a stirring device equipped with a stirring blade is used to stir the reaction liquid. At this time, since the stirring blade tip speed is 1.10 m/s or more, droplets of the organic phase and the aqueous phase suitable for the progress of the gas-liquid reaction are made fine, and similarly, bubbles of the gas phase are made fine. Since the stirring power per unit volume is 0.5 kW/$m^3$ or more and 3.5 kW/$m^3$ or less, the reaction liquid is sufficiently circulated. This enables contact between the liquid phase and the gas phase suitable for the progress of a gas-liquid reaction. In a case where the stirring power per unit volume is more than 3.5 kW/$m^3$ and the stirring blade tip speed is 1.10 m/s or more, the finer droplets of the liquid phase and the circulation of the liquid phase become dominant, and the frequency of contact among the liquid phases is increased more than the frequency of the gas-liquid contact, which tends to promote the reverse reaction for synthesizing the target organic compound. Therefore, this process is not suitable for improving yields. In a case where the stirring power per unit volume is less than 0.5 kW/$m^3$, the capacity to circulate the entire reaction liquid is insufficient, so the gas phase and the liquid phase cannot come into contact sufficiently with each other, and the yield tends to decrease. In a case where the stirring blade tip speed is less than 1.10 m/s, the droplets and the bubbles cannot be made sufficiently fine, so the gas phase and the liquid phase cannot come into sufficient contact with each other, and thus, the yield tends to decrease. Therefore, the method for producing an organic compound of the present embodiment can provide an organic compound with improved yields by satisfying the above-described stirring conditions.

In the method for producing an organic compound of the present embodiment, the reaction of the starting compound can be performed, for example, as follows. First, a gas phase and a reaction liquid including an organic phase and an aqueous phase are prepared. In the reaction liquid, the organic phase may include a catalyst and the aqueous phase may include the starting compound.

Next, a stirring device equipped with a reaction tank and a stirring blade is prepared, and the gas phase and the reaction liquid are introduced into the reaction tank.

The reaction is then performed by rotating the stirring blade to satisfy the stirring conditions, thereby stirring the reaction liquid. As an example, the starting compound reacts with the gas included in the gas phase. In a case where the gas includes hydrogen, the reaction of the starting compound is typically a hydrogenation reaction. It should be noted that the reaction of the starting compound is not limited to the hydrogenation reaction, but it may be a reduction reaction, a dehydration-condensation reaction, a hydrolysis reaction, or the like. In the method for producing an organic compound of the present embodiment, the gas phase may include hydrogen, and the hydrogenation reaction of the starting compound by the hydrogen may proceed.

The reaction of the starting compound is, for example, a reversible reaction. As mentioned above, the production method of the present embodiment tends to suppress progress of the reversible reaction by reducing the stirring power per unit volume to 3.5 kW/$m^3$ or less, while the reversible reaction is considered as one of the factors to decrease the yield. Therefore, the production method of the present embodiment can provide the effect of improvement in the yield particularly in a case where the reaction of the starting compound is a reversible reaction.

The stirring power per unit volume may be, for example, 3.3 kW/$m^3$ or less; it may be 3.0 kW/$m^3$ or less, 2.8 kW/$m^3$ or less, 2.5 kW/$m^3$ or less, 2.3 kW/$m^3$ or less, 2.0 kW/$m^3$ or less, 1.9 kW/$m^3$ or less, 1.8 kW/$m^3$ or less, 1.7 kW/$m^3$ or less, 1.6 kW/$m^3$ or less, 1.5 kW/$m^3$ or less, 1.4 kW/$m^3$ or less, 1.3 kW/$m^3$ or less, or even 1.2 kW/$m^3$ or less. The stirring power per unit volume may be, for example, 0.508 kW/$m^3$ or more; it may be 0.54 kW/$m^3$ or more, 0.6 kW/$m^3$ or more, 0.65 kW/$m^3$ or more, 0.7 kW/$m^3$ or more, 0.8 kW/$m^3$ or more, 0.9 kW/$m^3$ or more, or even 1.0 kW/$m^3$ or more. The stirring power per unit volume may be, for example, 0.5 kW/$m^3$ or more and 3.3 kW/$m^3$ or less, 0.508 kW/$m^3$ or more 3.0 kW/$m^3$ or less, 0.6 kW/$m^3$ or more and 2.8 kW/$m^3$ or less, 0.5 kW/$m^3$ or more and 1.8 kW/$m^3$ or less, 0.508 kW/$m^3$ or more and 1.5 kW/$m^3$ or less, or even 0.6 kW/$m^3$ or more and 1.5 kW/$m^3$ or less.

The stirring blade tip speed may be, for example, 1.20 m/s or more; it may be 1.30 m/s or more, 1.40 m/s or more, 1.50 m/s or more, 1.60 m/s or more, 1.70 m/s or more, 1.80 m/s or more, or even 1.90 m/s or more. The upper limit of the stirring blade tip speed is not particularly limited, but is, for example, 5.00 m/s or less.

The number of revolutions per minute of the stirring blade is not particularly limited, but may be, for example, 10 rpm or more; it may be 100 rpm or more, 200 rpm or more, 300 rpm or more, 400 rpm or more, 450 rpm or more, 485 rpm or more, 500 rpm or more, 600 rpm or more, 650 rpm or more, or even 670 rpm or more. The number of revolutions per minute of the stirring blade may be, for example, 1200 rpm or less; it may be 1100 rpm or less, 1000 rpm or less, 970 rpm or less, 900 rpm or less, 800 rpm or less, or even 750 rpm or less. The number of revolutions per minute of the stirring blade may be,

for example, 10 rpm or more and 1200 rpm or less, 100 rpm or more and 1100 rpm or less, or even 300 rpm or more and 1000 rpm or less.

The reaction liquid may be heated during the stirring. This will accelerate the synthesis reaction of the organic compound.

The temperature of the reaction liquid is not particularly limited, but the reaction liquid may be heated to, for example, 30°C or higher, 40°C or higher, 50°C or higher, 60°C or higher, or even 70°C or higher in order to efficiently promote the reaction. From the viewpoint of energy efficiency, preferably the temperature of the reaction liquid be 200°C or lower, 150°C or lower, 100°C or lower, lower than 100°C, or even 90°C or lower. The temperature of the reaction liquid in the reaction of starting compounds is, for example, 40°C or higher and 100°C or lower, 40°C or higher and lower than 100°C, 40°C or higher and 90°C or lower, or 65°C or higher and lower than 100°C. For example, the gas phase, the aqueous phase, and the organic phase may be heated to 90°C or to 70°C.

The time for the synthesis reaction is not particularly limited. In other words, the stirring time is not particularly limited. The reaction time is not particularly limited from the viewpoint of ensuring sufficient reactivity, but may be, for example, 0.5 hours or more; it may be 1 hour or more, 1.5 hours or more, 2 hours or more, 3 hours or more, 6 hours or more, 12 hours or more, 24 hours or more, 48 hours or more, 60 hours or more, or even 100 hours or more. The upper limit of the reaction time is not particularly limited, but may be, for example, 1000 hours or less, 800 hours or less, 300 hours or less, 200 hours or less, 100 hours or less, 50 hours or less, or even 20 hours or less.

The reaction pressure (pressure of gas in the reaction tank) is not particularly limited, but may be, for example, 0.1 MPa or more; may be 0.2 MPa or more, 0.5 MPa or more, 1 MPa or more, 4 MPa or more, 4.5 MPa or more, 5 MPa or more, or even 7 MPa or more from the viewpoint of improvement of the yield. The upper limit of the reaction pressure is not limited, and may be 50 MPa, for example; it may be 20 MPa, or even may be 10 MPa.

The resultant organic compound is dissolved in an aqueous phase, for example. After stirring, the organic phase and the aqueous phase may be collected, and the collected organic phase and the collected aqueous phase may be separated from each other. Since the aqueous phase and the organic phase can be separated by a simple process, the organic compound can be easily collected. In addition, expensive metal catalysts are likely to be reused without deactivation in their catalytic activity. By reusing the catalyst, high productivity can be achieved.

The production method of the present embodiment is suitable for improving the yield of organic compound in a reaction using a catalyst in the presence of a gas phase, an aqueous phase, and an organic phase. The yield is preferably 70% or more, and may be 75% or more, or even 80% or more. The upper limit of the yield is not particularly limited, and it is, for example, 99%.

The following is a detailed description of the stirring device, the reaction liquid, the gas phase, and the catalyst used in the method for producing an organic compound.

(Stirring device)

FIG. 1 is a cross-sectional view schematically showing an example of a stirring device to be used in the method for producing an organic compound of the present embodiment. The stirring device 100 includes a reaction tank 10 and a stirring blade 20, where the stirring blade 20 is attached to a stirring shaft 21. The reaction tank 10 contains a reaction liquid 30. The stirring blade 20 and the stirring shaft 21 may be integrated into a single unit. The stirring device 100 may further have one or more openings (not shown) for supplying the gas phase and the reaction liquid 30 (namely, organic phase and aqueous phase) to the interior.

The shape of the stirring blade 20 is not particularly limited. The stirring blade 20 can be, for example, an anchor blade, a turbine blade, a paddle blade, or blades collectively referred to as large blades. Examples of the large blades include FULLZONE (registered trademark) blade (KOBELCO ECO-SOLUTIONS CO., LTD.) and MAXBLEND (registered trademark) blade (Sumitomo Heavy Industries Process Equipment Co., Ltd.). The stirring blade 20 may be an anchor blade or a turbine blade.

The stirring blade diameter d is not particularly limited as long as the above-described stirring conditions are satisfied and the stirring blade 20 can be accommodated inside the reaction tank 10.

In the static state, a ratio h/L1, which is a ratio of a length h of the stirring blade 20 to a distance L1 from the inner bottom 10a of the reaction tank 10 to the liquid surface 30a of the reaction liquid 30 (i.e., the height of the liquid surface 30a in the reaction tank 10), may be 5% or more and 95% or less, or may be 5% or more and 50% or less. The length h of the stirring blade 20 is the blade length of the stirring blade 20 in the direction of the stirring shaft 21 (axial direction of rotation), as shown in FIG. 1. The stirring blade 20 may be an anchor blade and the ratio h/L1 may be 50% or more and 90% or less.

As shown in FIG. 2, in the static state, it is preferable that the interface 33 between the aqueous phase 31 and the organic phase 32 is in contact with the stirring blade 20. That is, in the static state, the distance L2 from the inner bottom 10a of the reaction tank 10 to the interface 33 is preferably larger than the distance from the inner bottom 10a of the reaction tank 10 to the bottom (one end in the axial direction) of the stirring blade 20 and smaller than the distance from the inner bottom 10a to the top (other end in the axial direction) of the stirring blade 20. It is more preferable that the proportion of the

organic phase in the reaction liquid 30 is 5 vol% or more and less than 50 vol% and that the interface 33 and the stirring blade 20 are in contact in the static state.

In the static state, a ratio L3 /L2 is preferably 0.5 or more and 1.8 or less, more preferably 0.7 or more and 1.5 or less, even more preferably 0.8 or more and 1.2 or less, and especially preferably 0.9 or more and 1.1 or less. Here, L2 is a distance from the inner bottom 10a of the reaction tank 10 to the interface 33 (i.e., the height of the interface 33 in the reaction tank 10), and L3 is a distance from the inner bottom 10a of the reaction tank 10 to the axial center 20c of the stirring blade 20 (i.e., the height of the axial center 20c of the stirring blade 20 in the reaction tank 10). The axial center 20c of the stirring blade 20 is the position at which the length h of the stirring blade 20 is divided into its half value $h_{1/2}$, as shown in FIG. 2. The axial direction typically coincides with the vertical direction. That is, the direction of the stirring shaft 21 typically coincides with the vertical direction. In the static state, the reaction tank 10 is arranged so that the liquid surface 30a and the interface 33 extend in the horizontal direction.

The volume of the reaction tank 10 is not particularly limited and can be selected according to the scale of production. The volume of the reaction tank 10 is, for example, 0.3 L or more and 10,000 L or less.

(Reaction liquid)

The reaction liquid 30 includes an aqueous phase and an organic phase.

The volume of the reaction liquid 30 is not particularly limited, and may be 0.05 L or more and 7000 L or less, may be 0.05 L or more and 5000 L or less, may be 0.05 L or more and 1000 L or less, may be 0.08 L or more and 500 L or less, may be 0.08 L or more and 100 L or less, may be 0.08 L or more and 50 L or less, may be 0.08 L or more and 20 L or less, may be 0.1 L or more and 20 L or less, may be 0.5 L or more and 20 L or less, may be 1.0 L or more and 20 L or less, may be 1.4 L or more and 20 L or less, may be 0.08 L or more and 1.4 L or less, or may be 1.4 L or more and 1,000 L or less.

The aqueous phase includes an aqueous solvent. The aqueous phase may further include a starting compound.

Examples of the aqueous solvents include water, methanol, ethanol, ethylene glycol, glycerin, and mixed solvents thereof. Among them, water is preferred from the viewpoint of a low load on the environment.

The aqueous phase may include an aqueous solvent and a starting compound.

Examples of the starting compounds include inorganic materials. Examples of the inorganic materials include carbon dioxide, a hydrogencarbonate, and a carbonate. That is, the starting compound may be at least one selected from the group consisting of carbon dioxide, a hydrogencarbonate, and a carbonate.

Examples of hydrogencarbonate and carbonate used in the present embodiment include a carbonate and a hydrogencarbonate of an alkali metal or an alkaline-earth metal. Examples of hydrogencarbonate include sodium hydrogencarbonate and potassium hydrogencarbonate. Potassium hydrogencarbonate is preferable from the viewpoint of high solubility with respect to water. That is, in the present embodiment, the starting compound preferably includes potassium hydrogencarbonate as the hydrogencarbonate. Examples of carbonate include sodium carbonate, potassium carbonate, potassium sodium carbonate, and sodium sesquicarbonate.

A hydrogencarbonate and a carbonate can be generated by reaction between a base and carbon dioxide. For example, a hydrogencarbonate or a carbonate may be generated by introducing carbon dioxide into a basic solution.

A solvent for the basic solution in generation of a hydrogencarbonate or a carbonate is not particularly limited, but may be water, methanol, ethanol, N,N-dimethylformamide, dimethyl sulfoxide, tetrahydrofuran, benzene, toluene, and mixed solvents thereof. The solvent preferably includes water, and is more preferably water. The base used for the basic solution is not particularly limited as long as the base can react with carbon dioxide to generate a hydrogencarbonate or a carbonate, and the base is preferably a hydroxide. Examples of the base include lithium hydrogencarbonate, sodium hydrogencarbonate, potassium hydrogencarbonate, cesium hydrogencarbonate, potassium hydroxide, sodium hydroxide, diazabicycloundecene, and triethylamine. Among them, the base is preferably a hydroxide, more preferably potassium hydroxide or sodium hydroxide, and even more preferably potassium hydroxide.

A content of the base in the basic solution is not particularly limited as long as a hydrogencarbonate and a carbonate can be produced. The content of the base is preferably 0.1 mol or more, more preferably 0.5 mol or more, and even more preferably 1 mol or more with respect to 1 L of the aqueous phase solvent from the viewpoint of ensuring an amount of generated formate. The content of the base is preferably 30 mol or less, more preferably 20 mol or less, and even more preferably 15 mol or less from the viewpoint of efficiency of the reaction. In a case where the content of the base is greater relative to the solubility of the aqueous phase, the solution may be suspended.

A ratio between carbon dioxide and the base in terms of amount to be used in the reaction between carbon dioxide and the base is preferably 0.1 or more, more preferably 0.5 or more, and even more preferably 1.0 or more as a molar ratio from the viewpoint of generating a carbonate from carbon dioxide. The ratio is preferably 8.0 or less, more preferably 5.0 or less, and even more preferably 3.0 or less from the viewpoint of utilization efficiency of carbon dioxide. The ratio between carbon dioxide and the base is a molar amount (mol) of $CO_2$/a molar amount (mol) of the base. In a case where the ratio between carbon dioxide and the base in terms of amount to be used is in the above-described range, carbon dioxide is inhibited from being excessively charged, and an amount of unreacted carbon dioxide can be minimized, so that the final

formic acid conversion efficiency is likely to be enhanced.

In the reaction for generating a hydrogencarbonate or a carbonate by reaction between carbon dioxide and the base, the reaction temperature is not particularly limited, but is preferably 0°C or higher, more preferably 10°C or higher, and even more preferably 20°C or higher in order to dissolve carbon dioxide in the aqueous phase. The reaction temperature is preferably 100°C or lower, more preferably 80°C or lower, and even more preferably 40°C or lower.

In the reaction for generating a hydrogencarbonate or a carbonate by reaction between carbon dioxide and the base, a reaction time is not particularly limited, but preferably is for example, 0.5 hours or more, more preferably one hour or more, and even more preferably two hours or more, from the viewpoint of sufficiently ensuring an amount of generated hydrogencarbonate or carbonate. The reaction time is preferably 24 hours or less, more preferably 12 hours or less, and even more preferably 6 hours or less from the viewpoint of cost.

The proportion of the aqueous phase in the reaction liquid 30 may be 40 vol% or more and 95 vol% or less, may be 45 vol% or more and 90 vol% or less, may be 50 vol% or more and 95 vol% or less, may be 50 vol% or more and 90 vol% or less, may be 50 vol% or more and 80 vol% or less, or may be 80 vol% or more and 95 vol% or less.

The organic phase includes an organic solvent. The organic phase may further include a catalyst. The organic phase preferably includes a catalyst and a solvent in which the catalyst is uniformly dissolved.

Examples of the organic solvent include toluene, benzene, xylene, propylene carbonate, dioxane, dimethyl sulfoxide, tetrahydrofuran, ethyl acetate, methyl cyclohexane, cyclopentyl methyl ether, and mixed solvents thereof, and from the viewpoint of separability from the aqueous solvent, the organic solvent preferably includes toluene or dioxane, and more preferably includes toluene.

The proportion of the organic phase in the reaction liquid 30 may be 5 vol% or more and 60 vol% or less, may be 5 vol% or more and 50 vol% or less, may be 10 vol% or more and 55 vol% or less, may be 10 vol% or more and 50 vol% or less, may be 20 vol% or more and 50 vol% or less, or may be 5 vol% or more and 20 vol% or less. According to the production method of the present embodiment, the organic compound can be produced in good yield regardless of the ratio of the organic phase.

(Gas phase)

The gas phase includes a gas. The gas phase may include at least a part of raw materials. The gas phase may include a gas capable of reacting with the starting compound. The gas may include, for example, hydrogen.

As a hydrogen supply source, for example, hydrogen generated in a smelting process in iron manufacture or hydrogen generated in a soda manufacturing process can be used. Hydrogen generated by electrolysis of water can also be utilized.

The gas phase may include a starting compound. For example, the gas phase may include a starting compound in a case where the starting compound is carbon dioxide.

Carbon dioxide may be a pure carbon dioxide gas, or may be a mixture with a component other than carbon dioxide. Examples of the component other than carbon dioxide include inert gases such as nitrogen and argon, water vapor, and any other component included in an exhaust gas and the like. Hydrogen and carbon dioxide may be used at the same proportion on a mole basis. However, hydrogen is preferably used in excess.

(Catalyst)

In the method for producing an organic compound of the present embodiment, the catalyst is, for example, a metal catalyst. The metal catalyst includes, for example, ruthenium.

In the method for producing an organic compound of the present embodiment, it is preferable that at least one compound selected from the group consisting of a metal complex represented by the following General formula (1A), a tautomer of the metal complex, a stereoisomer of the metal complex, and salts thereof is used as the catalyst.

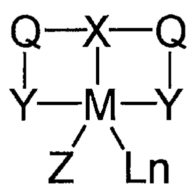

(1A)

(In General formula (1A),

X represents an atomic group including typical elements of Groups 13 to 15 that can coordinate to M,
each Q independently represents a cross-linked structure including typical elements of Groups 14 to 16 and linking Y

and X,
each Y independently represents an atomic group including typical elements of Groups 14 to 16 that can coordinate to M, and M represents a metal atom,
Z represents a halogen atom or a hydrogen atom,
n represents 0 to 3, and
in a case where the number of Ls is plural, each L independently represents a neutral or anionic ligand.)

In the description herein, the "Group n" means "Group n of the periodic table".

The typical elements of Groups 13 to 15 of the periodic table in X include boron, carbon, silicon, germanium, tin, nitrogen, phosphorus, arsenic, oxygen, sulfur, and selenium atoms; boron, carbon, silicon, germanium, tin, nitrogen, phosphorus, and arsenic atoms are preferable; carbon, nitrogen, phosphorus, and sulfur atoms are more preferable; and carbon or nitrogen atom is even more preferable.

X may be an atomic group with a valence of 0 to 1. Examples of the atomic group represented by X include an alkyl group, an alkenyl group, an alkoxy group, an aromatic ring, and a heterocyclic ring, and these may each have a substituent or may each combine with another substituent to form a ring.

Examples of the alkyl group in X include linear, branched, and cyclic substituted or unsubstituted alkyl groups. The alkyl group in X is preferably a C1 to C30 alkyl group, such as a methyl group, an ethyl group, an n-propyl group, an i-propyl group, a t-butyl group, an n-octyl group, an eicosyl group, and a 2-ethylhexyl group. The alkyl group in X is preferably an alkyl group in which the number of carbon atoms is 6 or less, and is preferably a methyl group.

Examples of the alkenyl group in X include linear, branched, and cyclic substituted or unsubstituted alkenyl groups. The alkenyl group in X is preferably a C2 to C30 alkenyl group, such as a vinyl group, an n-propyl group, an i-propenyl group, a t-butenyl group, and an n-octenyl group. The alkenyl group in X is preferably an alkenyl group in which the number of carbon atoms is 6 or less.

Examples of the alkoxy group in X include linear, branched, and cyclic substituted or unsubstituted alkoxy groups. The alkoxy group in X is preferably a C1 to C30 substituted or unsubstituted alkoxy group, such as a methoxy group, an ethoxy group, an isopropoxy group, a t-butoxy group, an n-octyloxy group, and a 2-methoxyethoxy group.

Examples of the aromatic ring in X include a phenyl ring and a naphthyl ring.

Examples of the heterocyclic ring in X include a pyrrolidine ring, a piperidine ring, a pyrroline ring, an imidazoline ring, an imidazolidine ring, a pyrrole ring, an imidazole ring, a pyridine ring, a pyrimidine ring, a triazine ring, a quinoline ring, and a quinazoline ring.

The atomic group with a valence of 0 to 1 represented by X preferably represents an atomic group including a heteroaromatic ring formed with two carbon atoms and a nitrogen atom, and may have a substituent, or may combine with another substituent to form a ring.

The atomic group with a valence of 0 to 1 represented by X is preferably a pyrroline ring, a pyridine ring, an imidazoline ring, a pyrimidine ring, or a triazine ring, is more preferably a pyridine ring or a triazine ring, and is even more preferably a pyridine ring.

In a case where the atomic group with a valence of 0 to 1 represented by X has a substituent, examples of the substituent include substituent group A, an alkyl group is preferable, and a methyl group is more preferable.

The cross-linked structure including typical elements of Groups 14 to 16 of the periodic table and linking Y and X represented by Q may have a double bond, may have a monocyclic structure or a condensed ring structure, and may have a substituent.

In Q, various structures can be introduced as described above, and the number of atoms in a portion between Y and X is, for example, preferably 1 to 5, more preferably 1 to 4, even more preferably 1 to 3, and particularly preferably 1 to 2.

Though the atoms in the portion between Y and X above is not particularly limited, carbon, nitrogen, phosphorus, oxygen, and sulfur atoms are preferable; carbon, nitrogen, and oxygen atoms are more preferable; carbon and oxygen atoms are even more preferable; and carbon atom is particularly preferable.

Q may have a monocyclic structure. In other words, the cross-linked structure represented by Q may include a ring structure.

In a case where Q has a monocyclic structure, the monocyclic structure may be directly bonded to Y and X in General formula (1A), or a divalent substituent may be sandwiched between the monocyclic structure and Y and/or Z in General formula (1A). Examples of the divalent substituent include a C1 to C5 alkylene group, a C2 to C5 alkenylene group, heteroatoms such as an oxygen atom and a sulfur atom, and any of these bonded in series.

It is preferable that each Q independently represents $CH_2$, NH, or O, $CH_2$ and NH may each further have a substituent, and it is more preferable that each Q represents $CH_2$ or NH.

Q may have a condensed ring structure. In other words, the cross-linked structure represented by Q may include a condensed ring structure.

In a case where Q has a condensed ring structure, the condensed ring structure may be directly bonded to Y and X

in General formula (1A), or a divalent substituent may be sandwiched between the condensed ring structure and Y and/or X in General formula (1A). The divalent substituent is the same as those described above as the divalent substituent sandwiched between the monocyclic structure and Y and/or X in General formula (1A).

Q may have a substituent. In a case where Q has neither a monocyclic structure nor a condensed ring structure, the substituent is a substituent in a Q portion in the ring structure configured to include Q, Y, X, and M in General formula (1A).

In a case where Q has a monocyclic structure or a condensed ring structure, the substituent is a substituent in the monocyclic structure or the condensed ring structure, or a substituent in another Q portion in the ring structure configured to include Q, Y, X, and M in General formula (1A).

The substituent that Q may have, for example, may have a heteroatom or may be another atom or atomic group.

Examples of the substituent having a heteroatom include a C1 to C18 alkoxy group, a C7 to C18 arylalkoxy group, a C6 to C18 aryloxy group, a C2 to C18 acyl group, a C7 to C18 aroyl group, a C2 to C18 dialkylamino group, an oxygen atom, and a sulfur atom.

Examples of the other atom or atomic group include a C3 to C18 aromatic group, a C1 to C18 alkyl group, and halogen atoms. Examples of aromatic groups include C6 to C20 aryl groups such as phenyl, xylyl, naphthyl, and biphenyl.

The number of carbon atoms in Q is preferably 12 or less, more preferably 10 or less, and even more preferably 8 or less.

Y may be an atomic group with a valence of 0 to 1. Each Y may independently represent an atomic group with a valence of 0 to 1 including typical elements of Groups 14 to 16 of the periodic table that can coordinate to M, and may further have a substituent. As the typical elements of Groups 14 to 16 of the periodic table, carbon, nitrogen, phosphorus, arsenic, oxygen, sulfur, and selenium atoms are preferable; carbon, nitrogen, phosphorus, and arsenic atoms are more preferable; nitrogen and phosphorus atoms are even more preferable; and a phosphorus atom is particularly preferable.

In General formula (1A), it is preferable that each Y represents a nitrogen atom or a phosphorus atom, or that one Y represents a phosphorus atom and the other Y represents a nitrogen atom.

In a case where the atomic group with a valence of 0 to 1 represented by Y has a substituent, examples of the substituent include substituent group A, an alkyl group or an aryl group is preferable, and an ethyl group, a t-butyl group, or a phenyl group is more preferable.

M represents a metal atom, and examples of M include elements of Groups 8 to 11 of the periodic table such as iron, ruthenium, osmium, cobalt, rhodium, iridium, nickel, palladium, platinum, copper, silver, and gold. Among them, iron, ruthenium, cobalt, iridium, nickel, palladium, or copper is preferable; ruthenium, rhodium, iridium, nickel, or palladium is more preferable; ruthenium, rhodium, iridium, or palladium is even more preferable; and ruthenium (Ru) is particularly preferable.

It is preferable that Z represents a halogen atom, and it is more preferable that Z represents a chlorine atom.

n represents an integer from 0 to 3 and represents the number of ligands coordinated to the metal atom represented by M. From the viewpoint of catalyst stability, n is preferably 2 or 3.

In a case where the number of Ls is plural, each L independently represents a neutral or anionic ligand.

Examples of the neutral ligand represented by L include ammonia, carbon monoxide, phosphines (e.g., triphenylphosphine, tris(4-methoxyphenyl)phosphine), phosphine oxides (e.g., triphenyl phosphine oxide), sulfides (e.g., dimethyl sulfide), sulfoxides (e.g., dimethyl sulfoxide), ethers (e.g., diethyl ether), nitriles (e.g., p-methylbenzonitrile), heterocyclic compounds (e.g., pyridine, N,N-dimethyl-4-aminopyridine, tetrahydrothiophene, and tetrahydrofuran), and the like, and preferably triphenylphosphine.

Examples of the anionic ligand represented by L include a hydride ion (hydrogen atom), a nitrate ion, and a cyanide ion, and the anionic ligand is preferably a hydride ion (hydrogen atom).

In General formula (1A), it is preferable that X represents a heterocyclic ring, each Q represents $CH_2$, NH, or O, each Y represents a phosphorus atom, and M represents ruthenium.

It is also preferable that Z represents a chlorine atom, n represents 1 to 3, and each L independently represents a hydrogen atom, carbon monoxide, or triphenylphosphine.

In the method for producing an organic compound of the present embodiment, the metal complex represented by General formula (1A) is preferably a metal complex represented by the following General formula (2A).

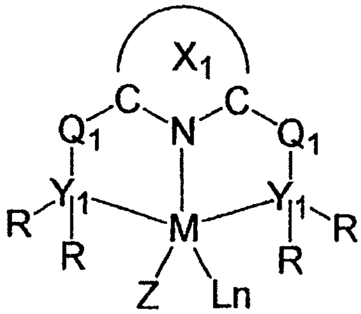

(2A)

(In General formula (2A),

$X_1$ represents a heteroaromatic ring formed with two carbon atoms and a nitrogen atom, and may have a substituent, or may combine with another substituent to form a ring,
each $Q_1$ independently represents $CH_2$, NH, or O, and $CH_2$ and NH may each further have a substituent,
each $Y_1$ independently represents a phosphorus atom or a nitrogen atom,
each R independently represents an alkyl group, an aryl group, or an aralkyl group, and may further have a substituent,
M represents a metal atom,
Z represents a halogen atom or a hydrogen atom,
n represents 0 to 3, and
in a case where the number of Ls is plural, each L independently represents a neutral or anionic ligand.)
M, $Q_1$, Z, n, and L in General formula (2A) are equivalent to M, Q, Z, n, and L in General formula (1A), respectively, and the preferable ranges are also the same.

The heteroaromatic ring formed with two carbon atoms and a nitrogen atom represented by $X_1$ is preferably a pyrroline ring, a pyridine ring, an imidazoline ring, a pyrimidine ring, or a triazine ring, is more preferably a pyridine ring or a triazine ring, and is even more preferably a pyridine ring.
Examples of the substituent that $X_1$ may have include substituent group A, an alkyl group is preferable, and a methyl group is more preferable.
Each $Y_1$ represents a phosphorus atom or a nitrogen atom, and each $Y_1$ may represent a nitrogen atom or a phosphorus atom, or one $Y_1$ may represent a phosphorus atom, and the other $Y_1$ may represent a nitrogen atom. It is preferable that each $Y_1$ is a nitrogen atom or a phosphorus atom, and it is even more preferable that each $Y_1$ is a nitrogen atom.
Examples of the alkyl group represented by R include linear, branched, and cyclic substituted or unsubstituted alkyl groups. The alkyl group represented by R is preferably a C1 to C30 alkyl group, such as a methyl group, an ethyl group, an n-propyl group, an i-propyl group, a t-butyl group, an n-octyl group, an eicosyl group, and a 2-ethylhexyl group. From the viewpoint of catalytic activity, an alkyl group in which the number of carbon atoms is 12 or less is preferable, an ethyl group or a t-butyl group is preferable, and a t-butyl group is more preferable.
The aryl group represented by R is a C6 to C30 substituted or unsubstituted aryl group, such as a phenyl group, a p-tolyl group, a naphthyl group, an m-chlorophenyl group, and an o-hexadecanoylaminophenyl group. An aryl group in which the number of carbon atoms is 12 or less is preferable, and a phenyl group is more preferable.
In a case where R further has a substituent, examples of the substituent include substituent group A, a methyl group, an ethyl group, an i-propyl group, a t-butyl group, or phenyl is preferable; and an ethyl group, an i-propyl group, or a t-butyl group is more preferable.
In General formula (2A), it is preferable that $X_1$ represents a pyridine ring or a triazine ring, each $Q_1$ represents $CH_2$, NH, or O, each $Y_1$ represents a phosphorus atom, each R represents an ethyl group, a t-butyl group, or a phenyl group, and M represents ruthenium.
It is also preferable that Z represents a chlorine atom, n represents 1 to 3, and each L independently represents a hydrogen atom, carbon monoxide, or triphenylphosphine.
In the method for producing an organic compound according to the first embodiment of the present invention, the metal complex represented by General formula (2A) is preferably a metal complex represented by the following General formula (3A).

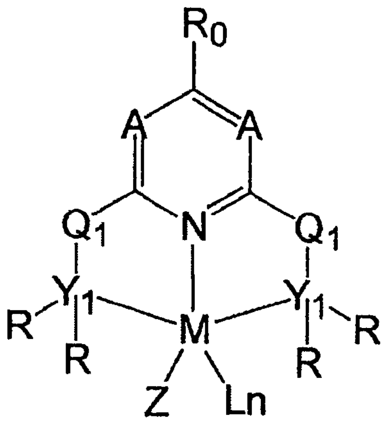

(3A)

(In General formula (3A),

$R_0$ represents a hydrogen atom or an alkyl group,
each A independently represents CH, $CR_5$, or N, and $R_5$ represents an alkyl group, an aryl group, an aralkyl group, an amino group, a hydroxy group, or an alkoxy group,
each $Q_1$ independently represents $CH_2$, NH, or O, and $CH_2$ and NH may each further have a substituent,
each $Y_1$ represents a phosphorus atom or a nitrogen atom,
each R independently represents an alkyl group, an aryl group, or an aralkyl group, and may further have a substituent,
M represents a metal atom,
Z represents a halogen atom or a hydrogen atom,
n represents 0 to 3, and
in a case where the number of Ls is plural, each L independently represents a neutral or anionic ligand.)
$Y_1$, R, $Q_1$, M, Z, n, and L in General formula (3A) are equivalent to $Y_1$, R, $Q_1$, M, Z, n, and L in General formula (2A), respectively, and the preferable ranges are also the same.

In General formula (3A), $R_0$ represents a hydrogen atom or an alkyl group. The alkyl group represented by $R_0$ is, for example, a linear, branched, or cyclic substituted or unsubstituted alkyl group. For example, the alkyl group represented by $R_0$ is preferably a C1 to C30 alkyl group, such as a methyl group, an ethyl group, an n-propyl group, an i-propyl group, a t-butyl group, an n-octyl group, an eicosyl group, and a 2-ethylhexyl group. An alkyl group in which the number of carbon atoms is 6 or less is preferable from the viewpoint of easily obtaining a raw material, and a methyl group is preferable.

In General formula (3A), $R_0$ is preferably a hydrogen atom or a methyl group.

Each A independently represents CH, $CR_5$, or N, and $R_5$ represents an alkyl group, an aryl group, an aralkyl group, an amino group, a hydroxy group, or an alkoxy group.

The alkyl group represented by $R_5$ is, for example, a linear, branched, or cyclic substituted or unsubstituted alkyl group. The alkyl group represented by $R_5$ is preferably a C1 to C30 alkyl group, such as a methyl group, an ethyl group, an n-propyl group, an i-propyl group, a t-butyl group, an n-octyl group, an eicosyl group, and a 2-ethylhexyl group. An alkyl group in which the number of carbon atoms is 12 or less is preferable from the viewpoint of easily obtaining a raw material, and a methyl group is preferable.

The aryl group represented by $R_5$ is, for example, a C6 to C30 substituted or unsubstituted aryl group, such as a phenyl group, a p-tolyl group, a naphthyl group, an m-chlorophenyl group, or an o-hexadecanoylaminophenyl group, and an aryl group in which the number of carbon atoms is 12 or less is preferable, and a phenyl group is more preferable.

The aralkyl group represented by $R_5$ is, for example, a substituted or unsubstituted aralkyl group in which the number of carbon atoms is 30 or less, and examples thereof include a trityl group, a benzyl group, a phenethyl group, a tritylmethyl group, a diphenylmethyl group, and a naphthylmethyl group, and an aralkyl group in which the number of carbon atoms is 12 or less is preferable.

The alkoxy group represented by $R_5$ is preferably a C1 to C30 substituted or unsubstituted alkoxy group, such as a methoxy group, an ethoxy group, an isopropoxy group, a t-butoxy group, an n-octyloxy group, and a 2-methoxyethoxy group.

In General formula (3A), it is preferable that $X_1$ represents a pyridine ring or a triazine ring, each $Q_1$ represents $CH_2$, NH, or O, each $Y_1$ represents a phosphorus atom, each R represents an ethyl group, a t-butyl group, or a phenyl group, and M represents ruthenium.

It is also preferable that Z represents a chlorine atom, n represents 1 to 3, and each L independently represents a hydrogen atom, carbon monoxide, or triphenylphosphine.

In the method for producing an organic compound of the present embodiment, the metal complex represented by General formula (3A) is preferably a ruthenium complex represented by the following General formula (4A).

The ruthenium complex represented by General formula (4A) is soluble in an organic solvent and insoluble in water, and is suitable as a catalyst in the production of organic compounds. The ruthenium complex represented by General formula (4A) is suitable, for example, as a catalyst in the production of formates. Since a formate generated by a reaction is easily soluble in water, a catalyst and the formate are easily separated by a reaction in a two-phase system, and each of the catalyst and the formate is easily separated and collected from the reaction system, so that it is possible to produce a formate at a high yield and the expensive catalyst is easily reused.

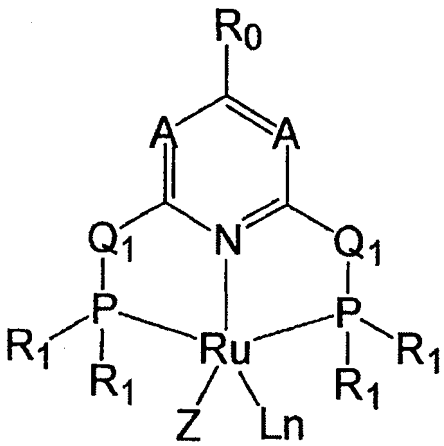

(4A)

(In General formula (4A),

$R_0$ represents a hydrogen atom or an alkyl group,
each $Q_1$ independently represents $CH_2$, NH, or O, and $CH_2$ and NH may each further have a substituent,
each $R_1$ independently represents an alkyl group or an aryl group (however, in a case where $Q_1$ represents NH or O, at least one $R_1$ represents an aryl group),
each A independently represents CH, $CR_5$, or N, and $R_5$ represents an alkyl group, an aryl group, an aralkyl group, an amino group, a hydroxy group, or an alkoxy group,
X represents a halogen atom,
n represents 0 to 3, and
in a case where the number of Ls is plural, each L independently represents a neutral or anionic ligand.)
$R_0$, A, $Q_1$, Z, L, and n in General formula (4A) are equivalent to $R_0$, A, $Q_1$, Z, L, and n in General formula (3A), respectively, and the preferable ranges are also the same.

The alkyl group and the aryl group represented by $R_1$ are equivalent to the alkyl group and the aryl group represented by R in General formula (3A), respectively, and the preferable ranges are also the same.

As for each of the metal complexes represented by General formula (1A) to General formula (4A), stereoisomers may be generated due to a coordination form or conformation of the ligands, but the metal complex may be a mixture of these stereoisomers, or may be a pure single isomer.

As the metal complexes represented by General formula (1A) to General formula (4A), complexes produced by a known method and the like can be used. As the known method, for example, a method described in E. Pidko et al., ChemCatChem 2014, 6, 1526-1530, etc., can be used.

Specific examples of the ruthenium complex represented by General formula (4A) include compounds illustrated below. In the compounds illustrated below, Et represents an ethyl group, tBu represents a tertiary butyl group, and Ph represents a phenyl group.

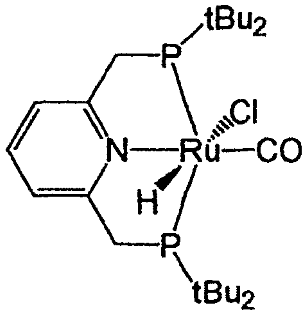

(1)

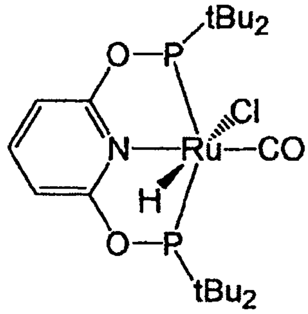

(2)

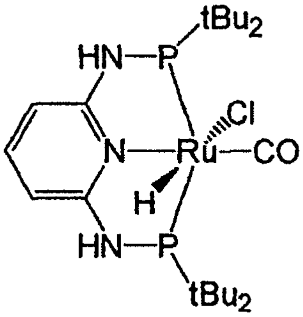

(3)

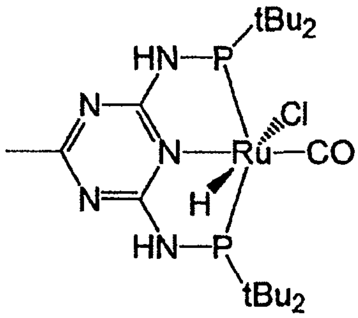

(4)

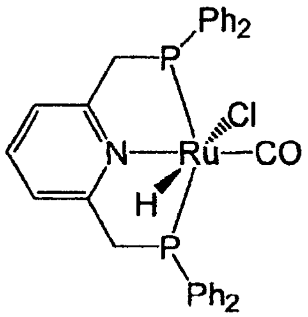

(5)

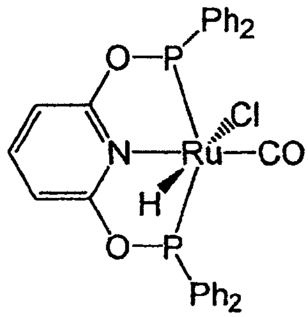

(6)

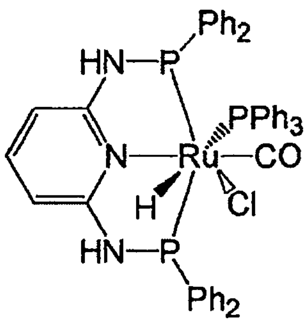

(7)

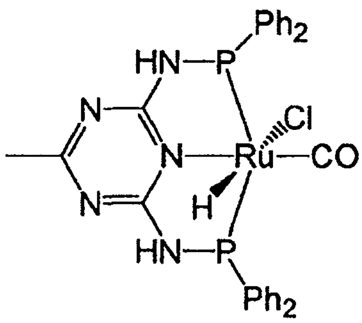

(8)

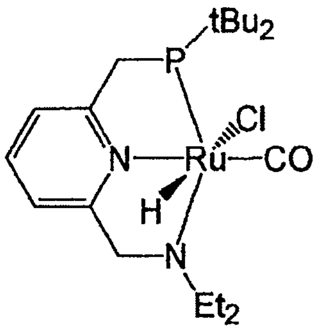

(9)

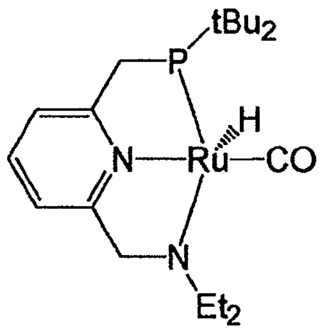

(10)

The amount of the catalyst (preferably, ruthenium complex) to be used is not particularly limited. From the viewpoint of sufficiently exhibiting the function of the catalyst, the amount of the catalyst to be used is preferably 0.1 μmol or more, more preferably 0.5 μmol or more, and even more preferably 1 μmol or more with respect to 1 L of the organic phase solvent, for example. From the viewpoint of cost, the amount of the catalyst to be used is preferably 1 mol or less, more preferably 10 mmol or less, and even more preferably 1 mmol or less with respect to 1 L of the organic phase solvent. Furthermore, from the viewpoint of suppressing a decrease in catalytic efficiency, the amount of the catalyst to be used may be 500 μmol or less, 400 μmol or less, or 100 μmol or less with respect to 1 L of the organic phase solvent. In a case where two or more kinds of catalysts are used, the total amount of the catalysts to be used may be in the above-described range.

(Phase transfer catalyst)

In the method for producing an organic compound of the present embodiment, a phase transfer catalyst for smoothly transferring substances between the aqueous phase and the organic phase may be used. Examples of the phase transfer catalyst include a quaternary ammonium salt, a quaternary phosphate, a macrocyclic polyether such as a crown ether, a nitrogen-containing macrocyclic polyether such as a cryptand, a nitrogen-containing linear polyether, and polyethylene glycol, and an alkyl ether thereof. Among them, a quaternary ammonium salt is preferred from the viewpoint of easily transferring substances between the aqueous solvent and the organic solvent even under a mild reaction condition.

Examples of the quaternary ammonium salt include methyltrioctylammonium chloride, benzyltrimethylammonium chloride, trimethylphenylammonium bromide, tributylammonium tribromide, tetrahexylammonium hydrogen sulfate, decyltrimethylammonium bromide, diallyldimethylammonium chloride, dodecyltrimethylammonium bromide, dimethyldioctadecylammonium bromide, tetraethylammonium tetrafluoroborate, ethyltrimethylammonium iodide, tris(2-hydroxyethyl)methylammonium hydroxide, tetramethylammonium acetate, tetramethylammonium bromide, and tetraethylammonium iodide. Methyltrioctylammonium chloride is preferable.

An amount of the phase transfer catalyst to be used is not particularly limited. The amount of the phase transfer catalyst to be used is preferably 0.1 mmol or more, more preferably 0.5 mmol or more, and even more preferably 1 mmol or more with respect to 1 L of the organic phase and the aqueous phase solvents. The amount of the phase transfer catalyst to be used is preferably 1 mol or less, more preferably 500 mmol or less, and even more preferably 100 mmol or less with respect to 1 L of the organic phase and the aqueous phase solvents from the viewpoint of cost. In a case where two or more kinds of phase transfer catalysts are used, the total amount of the phase transfer catalysts to be used may be in the above-described range.

(Other Ingredients)

In this production method, antioxidants may be added to the reaction liquid 30 as necessary. Examples of the antioxidants include a phosphorus-based antioxidant, an amine-based antioxidant, a phenol-based antioxidant, and a sulfur-based antioxidant. As such an antioxidant, for example, those disclosed in JP,2016-44190,A can be used. Additives such as UV absorbers and light stabilizers may be added to the reaction liquid 30 instead of or together with the antioxidants. These additives can be, for example, those disclosed in JP,2016-44190,A. The organic phase may include an antioxidant.

In a case of using an antioxidant, the amount of the antioxidant to be used is adjusted so that the antioxidant is soluble in the reaction liquid 30. From the viewpoint of sufficiently exhibiting the function of the antioxidant, the amount of the antioxidant to be used is preferably 1 mmol or more with respect to 1 L of the solvent. From the viewpoint of reducing the

cost of the antioxidant, the amount of the antioxidant to be used is preferably 100 mmol or less with respect to 1 L of the solvent. As the antioxidant, one kind may be used alone, or two or more kinds may be used in combination.

(Formate production method)

In the production method of the present embodiment, a formate may be synthesized by a hydrogenation reaction between hydrogen and at least one starting compound selected from the group consisting of carbon dioxide, a hydrogencarbonate, and a carbonate. The formate has a high hydrogen storage density, and it is safe and stable as a chemical substance, so that the formate can be easily handled, and hydrogen and carbon dioxide can be advantageously stored for a long time period. Formic acid can be generated by protonating at least a part of formate produced by the method for producing an organic compound of the present embodiment.

The resultant formate and formic acid have a wide range of applications in various fields, for example, as silage additives, feed preservatives, leather tanning agents, fiber dyes, rubber coagulants, anti-freeze agents, precision machinery cleaners, neutralizers, precipitants for heavy metals, de-icing agents, cutting fluids, heat transfer fluids, lubricants, hydride ion sources, and a source of hydrogen.

EXAMPLE

The present invention will be more specifically described below by way of Examples and Comparative Examples, though the present invention is not limited to the Examples. In each Example, a formate was synthesized by a hydrogenation reaction of a starting compound with hydrogen, using a catalyst, and the effect of improving the yield was evaluated.

[Synthesis of catalysts]

A catalyst was synthesized by the following operation. First, 40 mg (0.1 mmol) of the following ligand A was added to a THF (tetrahydrofuran) (5 ml) suspension of 95.3 mg (0.1 mmol) of $[RuHCl(PPh_3)_3(CO)]$ in an inert atmosphere, and the resultant mixture was stirred and heated at 65°C for three hours, thereby causing a reaction. Thereafter, the resultant product was cooled to room temperature (25°C). The thus obtained yellow solution was filtered, and the filtrate was evaporated to dryness under a vacuum. The obtained yellow residual oil was dissolved in a small amount of THF (1 mL), and hexane (10 mL) was slowly added to precipitate a yellow solid product, and the solid product was filtered. The filtered solid was dried under a vacuum, and the following catalyst 1 was obtained as yellow crystals. In the catalyst 1 and the ligand A shown below, tBu represents a tertiary butyl group.

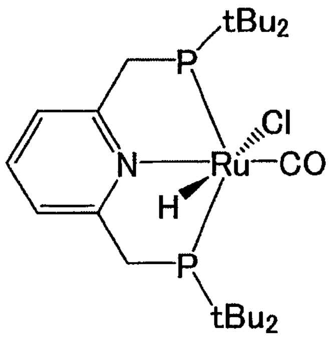

1

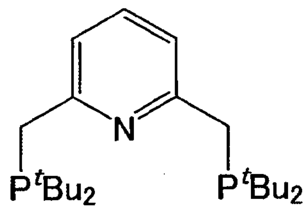

A

$^{31}P\{^1H\}(C_6D_6)$: 90.8(s), $^1H(C_6D_6)$: -14.54 (t, 1H, J = 20.0 Hz), 1.11 (t, 18H, J = 8.0 Hz), 1.51 (t, 18H, J = 8.0 Hz), 2.88 (dt, 2H, J = 16.0 Hz, J = 4.0 Hz), 3.76 (dt, 2H, J = 16.0 Hz, J = 4.0 Hz), 6.45 (d, 2H, J = 8.0 Hz), 6.79 (t, 1H, J = 8.0 Hz). $^{13}C\{^1H\}NMR(C_6D_6)$: 29.8(s), 30.7(s), 35.2 (t, J = 9.5 Hz), 37.7 (t, J = 6.0 Hz), 37.9 (t, J = 6.5 Hz), 119.5 (t, J = 4.5 Hz), 136.4(s), 163.4 (t, J = 5.0 Hz), 209.8(s).

[Calculation of yield]

In the following Examples 1 to 14 and Comparative Examples 1 to 2, the yield of a formate (potassium formate) was calculated by the following method.

First, the amount of substance of the formate included in the aqueous phase was determined as follows. To a 100

μL of aqueous phase discharged from a flow reactor, 100 μL of dimethyl sulfoxide as a reference substance was added, which was then dissolved in 500 μL of deuterium water. Thus, a measurement sample was prepared. On this measurement sample, $^1H$ NMR measurement performed. From the obtained NMR spectrum, an amount of substance X (mol) of the generated formate was calculated. Furthermore, the yield (%) of the formate was calculated by the following Formula (1) based on the amount of substance X (mol) of the generated formate, and the sum Z (mol) of amounts of substance of carbon dioxide, a hydrogencarbonate, and a carbonate used in the reaction (amount of substance of potassium hydrogencarbonate in the following Examples 1 to 14 and Comparative Examples 1 to 2).

$$\text{Yield of formate} = 100 \times X/Z \qquad (1)$$

[Example 1]

In a nitrogen gas atmosphere, 1.12 L of water, 4.7 mol of potassium hydrogencarbonate, 0.28 L of toluene, 70 μmol (250 μmol/L in an organic phase) of catalyst, and 15 mmol (54 mmol/L in an organic phase) of methyltrioctylammonium chloride were fed into a reactor equipped with an anchor blade. The stirring blade was installed to be positioned at the center of a reaction liquid. When the reactor was at rest, the interface of the organic phase and the aqueous phase was in contact with the stirring blade. Then, after feeding hydrogen gas up to 7 MPa, the temperature was raised to 90°C and stirring was performed under the stirring conditions shown in Table 1 until the reaction was completed. The reaction time for Example 1 was 224 minutes. The mixture was then cooled to room temperature and the pressure was carefully released after cooling. The inside of the reactor was replaced with a nitrogen gas.

In a nitrogen gas atmosphere, the reaction liquid was taken out from the reactor, and the organic phase and the aqueous phase were separated from each other. Using this aqueous phase, the yield of the formate was calculated by the above-described method.

[Example 2]

A reaction was performed in the same manner as in Example 1, except that the conditions were modified as shown in Table 1, and the yield was calculated.

[Example 3]

A reaction was performed in the same manner as in Example 1, except that the amounts of some components were changed as follows: 0.5 L of water, 2.1 mol of potassium hydrogencarbonate, 0.5 L of toluene, 30 μmol of catalyst (60 μmol/L in an organic phase), and 27 mmol of methyltrioctylammonium chloride (54 mmol/L in an organic phase) and that the conditions were modified as shown in Table 1, and the yield was calculated.

[Example 4]

A reaction was performed in the same manner as in Example 1, except that the amounts of the some components were modified as follows: 0.064 L of water, 0.42 mol of potassium hydrogencarbonate, 0.016 L of toluene, 4 μmol of catalyst (250 μmol/L in an organic phase), and 0.86 mmol of methyltrioctylammonium chloride (54 mmol/L in an organic phase) and that the conditions were modified as shown in Table 1, and the yield was calculated.

[Example 5]

A reaction was performed in the same manner as in Example 3, except that the conditions were modified as shown in Table 1, and the yield was calculated.

[Example 6]

A reaction was performed in the same manner as in Example 3, except that the stirring blade was changed to a disk turbine blade and that the conditions were modified as shown in Table 1, and the yield was calculated.

[Example 7]

A reaction was performed in the same manner as in Example 3, except that the stirring blade was changed to a disk turbine blade and that the conditions were modified as shown in Table 1, and the yield was calculated.

[Example 8]

A reaction was performed in the same manner as in Example 3, except that the stirring blade was changed to a disk turbine blade and that the conditions were modified as shown in Table 1, and the yield was calculated.

[Example 9]

A reaction was performed in the same manner as in Example 1, except that the stirring blade was changed to a disk turbine blade and that the conditions were modified as shown in Table 1, and the yield was calculated.

[Example 10]

A reaction was performed in the same manner as in Example 1, except that the stirring blade was changed to FULLZONE (registered trademark) blade (manufactured by KOBELCO ECO-SOLUTIONS) and that the conditions were modified as shown in Table 1, and the yield was calculated. The stirring blade was installed at a height that covers the entire height of the reaction solution.

[Example 11]

A reaction was performed in the same manner as in Example 1, except that the conditions were modified as shown in Table 1, and the yield was calculated.

[Example 12]

A reaction was performed in the same manner as in Example 1, except that the amounts of some components were changed as follows: 11.2 L of water, 56.0 mol of potassium hydrogencarbonate, 2.8 L of toluene, 700 μmol of catalyst (250 μmol/L in an organic phase), and 151 mmol of methyltrioctylammonium chloride (54 μmol/L in an organic phase) and that the conditions were modified as shown in Table 1, and the yield was calculated.

[Comparative Example 1]

A reaction was performed in the same manner as in Example 4, except that the amounts of water and toluene were changed to 0.08 L and 0.02 L respectively and that the conditions were modified as shown in Table 1, and the yield was calculated.

[Comparison Example 2]

A reaction was performed in the same manner as in Example 1, except that the stirring blade was changed to a FULLZONE (registered trademark) blade (manufactured by KOBELCO ECO-SOLUTIONS) and the conditions were modified as shown in Table 1, and the yield was calculated.

The results of Examples 1 to 12 and Comparative Examples 1 to 2 are shown in Table 1. In Table 1, the Froude number is a value calculated by a mathematical formula: stirring blade diameter d × number of revolutions $n^2$ / gravitational acceleration g. The reaction time is the time to be taken for the starting compound to complete the reaction (time at which equilibrium is reached).

FIG. 3 is a graph showing the stirring conditions for Examples 1 to 12 and Comparative Examples 1 to 2. In the plots in FIG. 3, the horizontal axis is the stirring power per unit volume (unit: $kw/m^3$) and the vertical axis is the stirring blade tip speed (unit: m/s). In FIG. 3, a marker "◦" indicates that the temperature of the reaction liquid during a reaction is 90°C and the yield is 80% or more; a marker "•" indicates that the temperature of the reaction liquid during a reaction is 70°C and the yield is 80% or more; a marker "◊" indicates that the yield is 75% or more and less than 80%, and a marker "×" indicates that the yield is less than 75%. The plots of Examples 6 and 7 overlap, where the marker "◊" refers to Example 6 and the marker "•" refers to Example 7.

[Table 1]

| | Example 1 | Example 2 | Example 3 | Example 4 | Example 5 | Example 6 | Example 7 |
|---|---|---|---|---|---|---|---|
| Reaction liquid volume [L] | 1.4 | 1.4 | 1.0 | 0.08 | 1.0 | 1.0 | 1.0 |

(continued)

| | Example 1 | Example 2 | Example 3 | Example 4 | Example 5 | Example 6 | Example 7 |
|---|---|---|---|---|---|---|---|
| Organic phase proportion [vol%] | 20 | 20 | 50 | 20 | 50 | 50 | 50 |
| Hydrogen pressure [MPa] | 7 | 7 | 7 | 5 | 5 | 7 | 7 |
| Reaction liquid temperature [°C] | 90 | 90 | 70 | 90 | 90 | 90 | 70 |
| Stirring blade type | Anchor blade | Anchor blade | Anchor blade | Anchor blade | Anchor blade | Disk turbine blade | Disk turbine blade |
| Number of revolutions of stirring blade [rpm] | 670 | 750 | 600 | 970 | 485 | 485 | 485 |
| Stirring power per unit volume [kW/m$^3$] | 1.054 | 1.478 | 1.02 | 1.147 | 0.54 | 0.68 | 0.68 |
| Froude number | 0.70 | 0.88 | 0.56 | 1.47 | 0.37 | 0.37 | 0.37 |
| Stirring blade tip speed [m/s] | 1.93 | 2.16 | 1.73 | 1.32 | 1.40 | 1.40 | 1.40 |
| Reaction time [min] | 224 | 120 | 104 | 170 | 115 | 65 | 783 |
| Yield [%] | 87 | 84 | 83 | 77 | 75 | 79 | 81 |

[Table 1] Continued

| | Example 8 | Example 9 | Example 10 | Example 11 | Example 12 | Comparative Example 1 | Comparative Example 2 |
|---|---|---|---|---|---|---|---|
| Reaction liquid volume [L] | 1.0 | 1.4 | 1.4 | 1.4 | 14 | 0.1 | 1.4 |
| Organic phase proportion [vol%] | 50 | 20 | 20 | 20 | 20 | 20 | 20 |
| Hydrogen pressure [MPa] | 5 | 5 | 7 | 7 | 5 | 5 | 5 |
| Reaction liquid temperature [°C] | 90 | 90 | 90 | 90 | 90 | 90 | 90 |
| Stirring blade type | Disk turbine blade | Disk turbine blade | FULLZONE blade | Anchor blade | Anchor blade | Anchor blade | FULLZONE blade |
| Number of revolutions of stirring blade [rpm] | 600 | 485 | 690 | 570 | 375 | 800 | 750 |
| Stirring power per unit volume [kW/m$^3$] | 1.282 | 0.508 | 2.79 | 0.79 | 1.16 | 0.531 | 3.59 |
| Froude number | 0.56 | 0.37 | 0.74 | 0.51 | 0.22 | 1.00 | 0.88 |

(continued)

| | Example 8 | Example 9 | Example 10 | Example 11 | Example 12 | Comparative Example 1 | Comparative Example 2 |
|---|---|---|---|---|---|---|---|
| Stirring blade tip speed [m/s] | 1.73 | 1.40 | 1.99 | 1.64 | 2.45 | 1.09 | 2.16 |
| Reaction time [min] | 692 | 934 | 77 | 70 | 65 | 170 | 62 |
| Yield [%] | 79 | 76 | 86 | 78 | 89 | 63 | 69 |

Examples 1 to 12 satisfying the stirring conditions of stirring power per unit volume of 0.5 kW/$m^3$ or more and 3.5 kW/$m^3$ or less and stirring blade tip speed of 1.1 m/s or more showed higher yields in comparison with Comparative Examples 1 to 2. Table 1 shows that in a case where the reaction liquid temperature is the same, a higher stirring blade tip speed tends to result in a higher yield.

[Example 13]

A reaction was performed in the same manner as in Example 1, except that the conditions were modified as shown in Table 2, and the yield was calculated.

[Example 14]

A reaction was performed in the same manner as in Example 13, except that the position (height) of the stirring blade was changed as shown in Table 2, and the yield was calculated.

The results of Examples 10 and 13 to 14 are shown in Table 2. In Table 2, "Reaction liquid surface height" is a distance L1 from the inner bottom of the reactor (reaction tank) to the liquid surface of the reaction liquid. "Aqueous-organic phase interface height" is a distance L2 from the inner bottom of the reactor (reaction tank) to the liquid surface of the interface between the aqueous phase and the organic phase. "Ratio L3/L2" is a ratio of a distance L3 to the distance L2, where the distance L3 is a distance from the inner bottom of the reactor (reaction tank) to the axial center of the stirring blade. "Stirring blade bottom height" is a distance from the inner bottom of the reactor (reaction tank) to the bottom of the stirring blade. "Yield 80% reach time" is the time at which the absolute value of the formate yield reaches 80%.

[Table 2]

| | Example 10 | Example 13 | Example 14 |
|---|---|---|---|
| Reaction liquid volume [L] | 1.4 | 1.4 | 1.4 |
| Organic phase proportion [vol%] | 20 | 20 | 20 |
| Hydrogen pressure [MPa] | 7 | 7 | 7 |
| Reaction liquid temperature [°C] | 90 | 90 | 90 |
| Stirring blade type | FULLZONE blade | Anchor blade | Anchor blade |
| Stirring blade installation position | Entire reaction liquid | Water-toluene interface | Reaction liquid center |
| Contact between aqueous-organic phase and stirring blade | Yes | Yes | No |
| Reaction liquid surface height [mm] | 215 | 215 | 215 |
| Aqueous-organic phase interface height [mm] | 176 | 176 | 176 |
| Stirring blade bottom height [mm] | 40 | 162 | 93.5 |
| Ratio L3/L2 | 1.3 | 1 | 1.6 |
| Number of revolutions of stirring blade [rpm] | 690 | 750 | 750 |
| Stirring power per unit volume [kW/$m^3$] | 2.79 | 1.76 | 1.76 |

(continued)

| | Example 10 | Example 13 | Example 14 |
|---|---|---|---|
| Froude number | 0.74 | 0.88 | 0.88 |
| Stirring blade tip speed [m/s] | 1.99 | 2.16 | 2.16 |
| Reaction time [min] | 77 | 37 | 140 |
| Yield 80% reach time [min] | 43 | 29 | 81 |
| Yield [%] | 85.8 | 83 | 85 |

The yield 80% reach time was shorter in Example 13 than in Example 14. This result shows that the reaction rate tends to increase when the position of the stirring blade is set so that the stirring blade and the interface of the aqueous phase and the organic phase is in contact while the reactor is at rest. This is presumably because the area of the interface between the organic phase and the gas phase during stirring is increased, and contact between the gas phase and the liquid phase suitable for the reaction is achieved. The results of Example 10 and Example 13 also show that adjustment of the installation position of the stirring blade can improve the reaction rate without increasing the stirring power per unit volume. These results suggest that even an anchor blade can provide results equal to or better than the results provided by a FULLZONE blade, the results including gas-liquid dispersion and contact between the gas phase and the liquid phase.

INDUSTRIAL APPLICABILITY

According to the method for producing an organic compound of the present embodiment, for example, a target organic compound can be produced in an improved yield.

**Claims**

1. A method for producing an organic compound by reacting a starting compound using a catalyst in the presence of a gas phase and a liquid phase, the liquid phase including an organic phase and an aqueous phase,
the method comprising:
stirring the liquid phase under stirring conditions where a stirring power per unit volume is 0.5 kW/m$^3$ or more and 3.5 kW/m$^3$ or less and a stirring blade tip speed is 1.10 m/s or more.

2. The method according to claim 1, wherein the stirring blade tip speed is 1.8 m/s or more.

3. The method according to claim 1, wherein the stirring power per unit volume is 2.8 kW/m$^3$ or less.

4. The method according to claim 1, wherein under the stirring conditions, the number of revolutions is 10 rpm or more and 1200 rpm or less.

5. The method according to claim 1, wherein the liquid phase is 0.05 L or more and 7000 L or less.

6. The method according to claim 1, wherein a proportion of the organic phase in the liquid phase is 5 vol% or more and 60 vol% or less.

7. The method according to claim 1, wherein the liquid phase has a temperature of 40°C or higher and 100°C or lower in the reaction.

8. The method according to claim 1, wherein

    the stirring is performed using a stirring blade, and
    in a static state, the stirring blade is in contact with an interface between the aqueous phase and the organic phase.

9. The method according to claim 1, wherein the reaction is a reversible reaction.

10. The method according to claim 1, wherein the organic phase includes the catalyst and the aqueous phase includes the

starting compound.

**11.** The method according to claim 1, wherein the gas phase includes a gas capable of reacting with the starting compound.

**12.** The method according to claim 11, wherein the gas includes hydrogen, and
the reaction is a hydrogenation reaction of the starting compound.

**13.** The method according to claim 12, wherein the starting compound is at least one selected from the group consisting of carbon dioxide, a hydrogencarbonate and a carbonate, and a formate is synthesized from the starting compound by the reaction.

**14.** The method according to claim 1, wherein the catalyst is a metal catalyst.

**15.** The method according to claim 14, wherein the metal catalyst includes ruthenium.

**16.** The method according to claim 1, wherein the organic phase includes toluene.

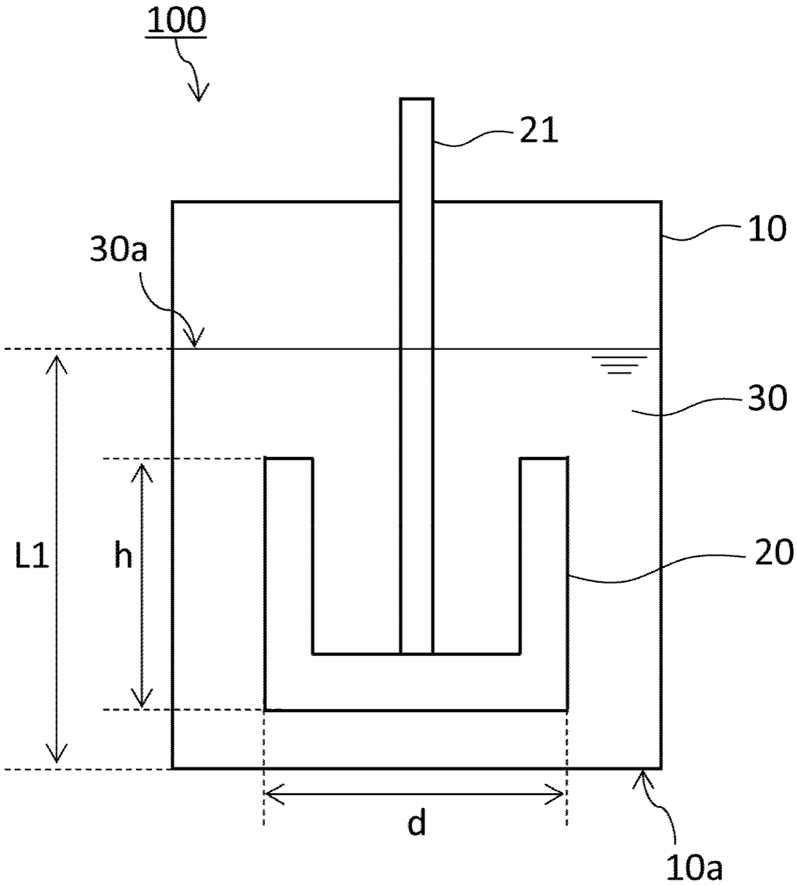


FIG.1

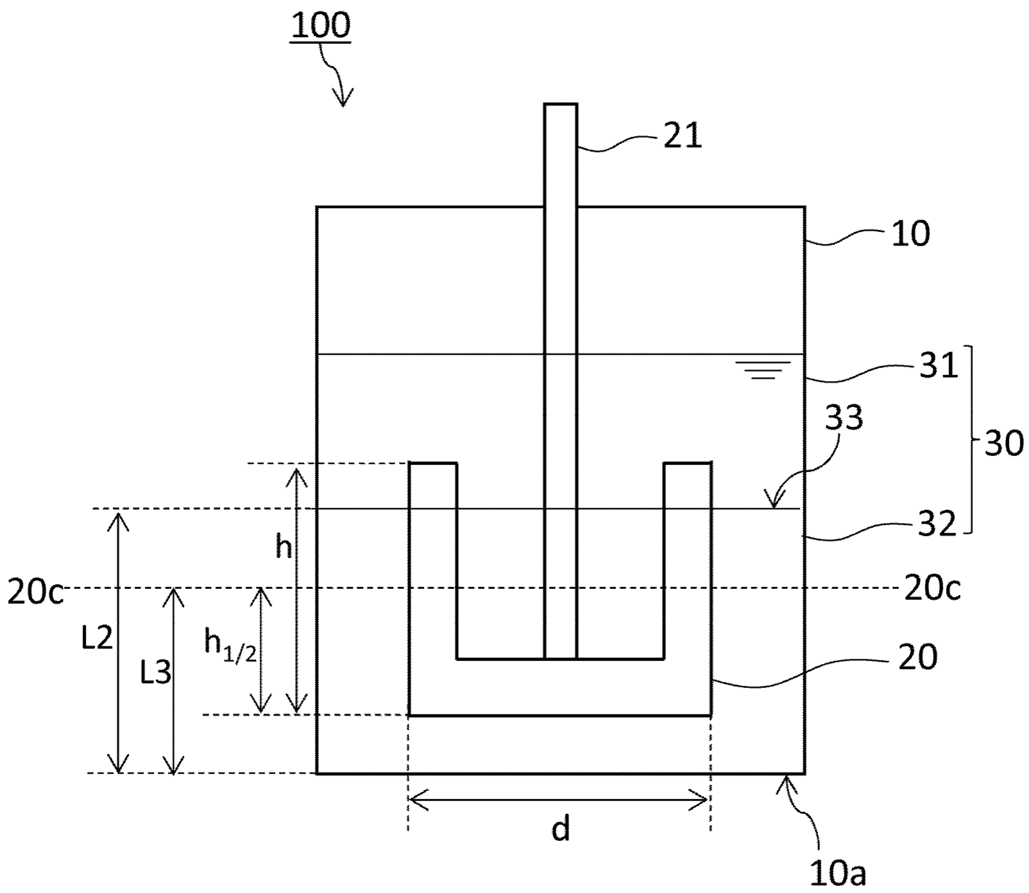


FIG.2

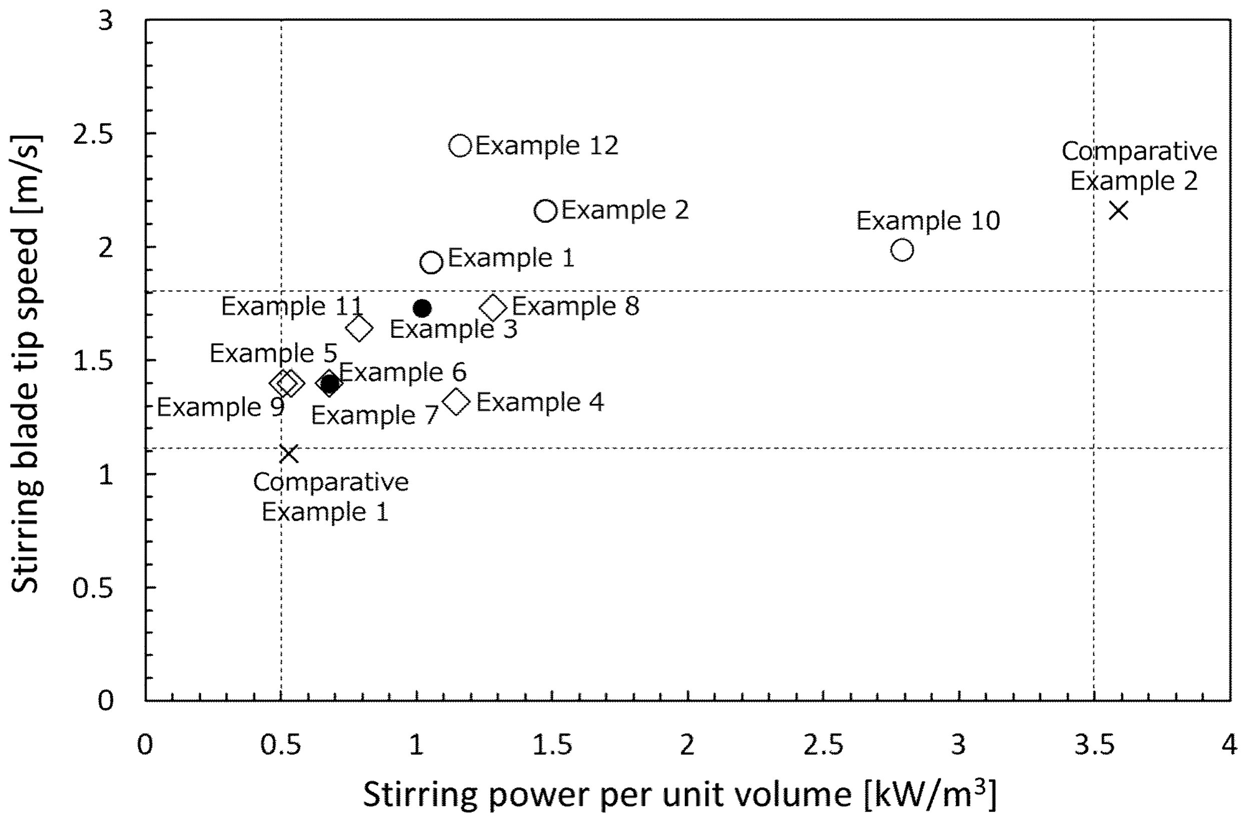
Stirring blade tip speed [m/s]
Stirring power per unit volume [kW/m3]
0
0.5
1
1.5
2
2.5
3
3.5
4
Example 12
Example 2
Example 1
Example 10
Comparative Example 2
Example 11
Example 3
Example 8
Example 5
Example 6
Example 9
Example 7
Example 4
Comparative Example 1

FIG.3

**INTERNATIONAL SEARCH REPORT**

International application No. **PCT/JP2024/006111**

**A. CLASSIFICATION OF SUBJECT MATTER**

***C07C 51/00***(2006.01)i; ***B01F 23/43***(2022.01)i; ***B01F 23/231***(2022.01)i; ***B01F 27/90***(2022.01)i; ***B01F 27/1122***(2022.01)i; ***B01F 33/71***(2022.01)i; ***B01F 35/221***(2022.01)i; ***B01F 35/222***(2022.01)i; ***B01J 31/24***(2006.01)i; ***C07B 61/00***(2006.01)i; ***C07C 53/06***(2006.01)i

FI: C07C51/00; C07C53/06; B01J31/24 Z; B01F27/1122; B01F27/90; B01F33/71; B01F35/222; B01F35/221; B01F23/43; B01F23/231; C07B61/00 300

According to International Patent Classification (IPC) or to both national classification and IPC

**B. FIELDS SEARCHED**

Minimum documentation searched (classification system followed by classification symbols)

C07C51/00; B01F23/43; B01F23/231; B01F27/90; B01F27/1122; B01F33/71; B01F35/221; B01F35/222; B01J31/24; C07B61/00; C07C53/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2022/050236 A1 (NITTO DENKO CORPORATION) 10 March 2022 (2022-03-10) entire text, all drawings | 1-16 |
| A | WO 2022/050235 A1 (NITTO DENKO CORPORATION) 10 March 2022 (2022-03-10) entire text, all drawings | 1-16 |
| A | JP 2006-55847 A (BAYER MATERIALSCIENCE AKTIENGESELLSCHAFT) 02 March 2006 (2006-03-02) entire text, all drawings | 1-16 |
| A | JP 2000-128850 A (SUMITOMO CHEMICAL CO., LTD.) 09 May 2000 (2000-05-09) entire text, all drawings | 1-16 |
| A | JP 2013-530195 A (BASF SE) 25 July 2013 (2013-07-25) entire text, all drawings | 1-16 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 April 2024** | **14 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/006111**

| Patent document cited in search report | Publication date (day/month/year) | Patent family member(s) | Publication date (day/month/year) |
|---|---|---|---|
| WO 2022/050236 A1 | 10 March 2022 | US 2024/0043367 A1<br>entire text, all drawings<br>US 2023/0312448 A1<br>US 2023/0357120 A1<br>EP 4209480 A1<br>EP 4209478 A1<br>EP 4209479 A1<br>CN 116075494 A<br>CN 116096698 A<br>CN 116096699 A | |
| WO 2022/050235 A1 | 10 March 2022 | US 2023/0312448 A1<br>entire text, all drawings<br>US 2023/0357120 A1<br>US 2024/0043367 A1<br>EP 4209479 A1<br>EP 4209478 A1<br>EP 4209480 A1<br>CN 116096698 A<br>CN 116075494 A<br>CN 116096699 A | |
| JP 2006-55847 A | 02 March 2006 | US 2006/0038306 A1<br>entire text, all drawings<br>EP 1637220 A1<br>KR 10-2006-0050500 A<br>CN 1915480 A | |
| JP 2000-128850 A | 09 May 2000 | US 6603035 B1<br>entire text, all drawings | |
| JP 2013-530195 A | 25 July 2013 | WO 2012/000823 A1<br>entire text, all drawings<br>EP 2588439 A1<br>CN 103080061 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000128850 A
- JP 2016044190 A


### Non-patent literature cited in the description


- **E. PIDKO et al.** *ChemCatChem*, 2014, vol. 6, 1526-1530